(19) Europäisches Patentamt
European Patent Office
Office européen des brevets

(11) Publication number: **0 234 095**
A2

(12) # EUROPEAN PATENT APPLICATION

(21) Application number: **86308281.4**

(22) Date of filing: **24.10.86**

(51) Int. Cl.³: **C 12 Q 1/48**
//C12N5/00, C12N15/00, C12P21/00

(30) Priority: **24.10.85 US 790813**
**12.11.85 US 796904**
**09.01.86 US 817433**
**11.06.86 US 873199**

(43) Date of publication of application:
**02.09.87 Bulletin 87/36**

(84) Designated Contracting States:
**AT BE CH DE ES FR GB IT LI LU NL SE**

(71) Applicant: **BIOTECHNOLOGY RESEARCH PARTNERS LIMITED**
**2450 Bayshore Parkway**
**Mountain View, California 94043(US)**

(72) Inventor: **Shine, John**
**406 Deodara Drive**
**Los Alto California 94022(US)**

(72) Inventor: **Baker, Andrew R.**
**863 Warren Way**
**Palo Alto California 94303(US)**

(72) Inventor: **Frossard, Philippe M.**
**580 Arastradero Road Apt. 304**
**Palo Alto California 94306(US)**

(74) Representative: **Goldin, Douglas Michael et al,**
**J.A. KEMP & CO. 14, South Square Gray's Inn**
**London WC1R 5EU(GB)**

(54) **Blood tests diagnostic for hypertension.**

(57) Polymorphisms in the renin, kallikrein, and ANF gene regions are predictive for hypertension in individual human subjects, and are useful for genetic analysis. DNA encoding human kallikrein provides a probe for detection of these polymorphisms and permits both production of recombinant protein in practical quantities and manipulation of the amino acid sequence to optimize properties.

EP 0 234 095 A2

## BLOOD TESTS DIAGNOSTIC FOR HYPERTENSION

### Technical Field

The invention relates to the use of genetic polymorphism detection to diagnose presence of, or probability of, disease, and to the cloning and expression of a gene useful in detecting these polymorphisms and in the treatment of the corresponding disease. More particularly, it relates to the use of these techniques to assess the risk of, and follow familial patterns of, the incidence of hypertension, and to the cloning and expression of the gene for human kallikrein.

### Background Art

Hypertension is one of the most important public health problems in developed countries. It affects more than 60 million people in the United States alone. The disease, which is defined as an elevation of arterial blood pressure, results in secondary organ damage and a reduced lifespan. It occurs in two forms. Essential hypertension, also called primary hypertension, accounts for 95% of all cases, and is of unknown cause. Secondary hypertension, which constitutes the remaining 5% of the cases found, is associated with known causes, such as primary hyperaldosterism, renal disease, or coarctation of the aorta.

While no direct cause for the majority of hypertension cases has been found, some of the metabolic factors which regulate blood pressure are known. Notable among these are the angiotensin and kallikrein systems, which control vasoconstriction and dilation, and the salt-regulating system, which controls the levels of sodium ion in the bloodstream. The angiotensin system contains a number of critical proteins, prominent among which is renin, a key enzyme in the production of an octapeptide, angiotensin-II, which acts directly to constrict vascular smooth muscles and which also induces the release of aldosterone from the adrenal cortex. The proteins of the kallikrein system influence the level of kinin, a vasodilator. The levels of sodium ion are, on the other hand, regulated by an atrial natriuretic peptide factor (ANF). Renin and ANF are human proteins of determined amino acid sequence. The genes encoding these proteins have been cloned and expressed, the cloning and expression of the kallikrein gene is disclosed herein.

There is, at present, no predictive diagnosis for hypertension, nor is there any accurate diagnosis for the various causes of hypertension. It is singularly important to have a predictive tool in the case of this disease, as various preventive therapies, based, for example, on control of the above-mentioned factors, are available. In addition, in relatively benign cases, even regulation of diet, lifestyle, or other noninvasive practices are effective in minimizing risk.

A technique that inherently offers the advantages of early detection, if its results can be effectively correlated with the disorder to be assessed, is genetic analysis. Since the genomic characteristics of an individual are basically determined, it is assumed, at conception, genetic aberrations which are indicia of later metabolic disorders are an ideal early diagnosis tool. Genetic testing can be routinely conducted using present methodology, as early as the seventh week of fetal life. In adults and children, it can be accomplished using a small blood sample. Over the last ten years, the availability of restriction enzymes and DNA probing techniques has made possible the use of "restriction fragment length polymorphisms" (RFLPs) in such diagnosis. Using the, by now, well established Southern blot hybridization technique (Southern, E., J Mol Biol (1975) 98:503-517), it has been possible successfully to diagnose sickle cell anemia (Kan, Y.W., et al, Proc Natl Acad Sci (USA) (1978) 75:5631); ß-thalassemia (Antonarakis, S.E., et al, Proc Natl Acad Sci (USA) (1983) 79:137); type II diabetes (Rotwein, P., et al, Science (1981) 213:1117); familial growth hormone deficiency (Phillips, J.A., III, Banbury Report 14, Cold Spring Harbor Laboratory (1983) pp 305-315); phenylketonuria (Woo, S.L.C., et al, Nature (1983) 306:151); Huntington's disease (Gusella, J.F., et al, Nature (1983) 306:234); and hemophilia B (Gianelli, et al, Lancet (1984) i:239, Grunenbaum, et al, J Clin Invest (1984) 73:1491).

The use of genetic detection systems is particularly appropriate in predicting the incidence of hypertension for an individual since it appears that genetic, rather than environmental, factors are in large part responsible for the onset of the disease. This has been shown in both animal and human studies, including of blood pressures which exist as patterns or "aggregations" within families. Adopted children do not demonstrate familial aggregation of blood pressures. Therefore, an assessment of the genome of the subject provides fertile ground for assessment of risk.

All of the successful predictive tests listed above are grounded in identification of a particular polymorphism or polymorphisms which correlates with the disease or disorder in question. It has been calculated that the number of polymorphisms expected in the human genome should be of the order of $10^7$, based on an assumed probability of 0.005 for a given nucleotide to be polymorphic; this number being inferred from studies of the human growth hormone, αI-antitrypsin and ß-like globin gene cluster loci (Jeffreys, A. J., Cell (1979) 18:1-10; Oster, H., et al, Am J Hum Gen (1984) 36(suppl) 150S). The challenge is to determine which of these over ten million polymorphisms is associated with a particular disorder, and to devise a procedure to detect it.

The present invention uses the gene loci associated with the production of renin, of ANF, or of kallikrein as sites for the occurrence of polymorphisms associated with hypertension.

The availability of the appropriate DNA sequences has made this a feasible approach. Human renin cDNA and genomic DNA have been described by Hardmann, J.A., et al, DNA (1984) 3:457; Imai, T., et al, Proc Natl Acad Sci (USA) (1983) 80: 7405; Hobart, P.M., et al, Proc Natl Acad Sci (USA) (1984) 81: 5026; Miyazaki, H., et al, Proc Natl Acad Sci (USA) (1984) 81: 5999. DNA encoding human ANF has been described by Greenberg, B.D., et al, Nature (1984) 312:656-658; Yamanaka, N., et al, Nature (1984) 309: 719; Maki, M., et al, Nature (1984) 309: 722; Oikawa, S., et al, Nature (1984) 309: 724; Seidman, C.E., et al, Science (1984) 225: 324; Zivin, R.A., et al, Proc Natl Acad Sci (USA) (1984) 81: 6325. The cDNA sequence encoding human kallikrein is disclosed hereinbelow.

Kallikrein, isolated from human urine, has been of considerable medical use in the treatment of cerebro- and cardiovascular disorders, including cardioartero- sclerosis and hypertension. While human kallikrein has been isolated from this source in sufficient quantity and purity to serve this purpose, it is, of course, extremely expensive, and is also relatively unstable, and must be treated for use in medical protocols with stabilizing agents, for example with human serum albumin. Recombinant production of this protein would provide large amounts of inexpensive pure material. The availability of the DNA sequence encoding this protein also permits convenient means for modifying the amino acid sequence to provide the needed stability without reducing activity, or to enhance the biological potency of this material; in particular, however, the availability of this sequence permits the detection of additional polymorphisms useful in diagnosing hypertension.

The present invention provides a number of polymorphic sites in the above-mentioned gene regions which are useful both alone and in combination in predicting the incidence of hypertension by means of a simple blood test.

## Disclosure of the Invention

The invention provides identification of polymorphisms which are predictive of the subsequent development of hypertension and others which are indicative of an individual protected against development of this disease. These polymorphisms are found in the regions of the genome which encodes renin, ANF, and kallikrein. Taken together or in subsets, they provide an identity profile of a particular individual, thus providing a means to follow family inheritance patterns and to assess relationships between individuals in accordance with the familial aggregation of this symptomology. In addition, since these polymorphisms are located in the genomic sequences which regulate blood pressure, is is probable that their pattern of presence or absence will show some additional specific, as yet unknown, correlation to symptomology.

The invention is also directed to DNA sequences encoding human kallikrein, to expression systems which are capable of effecting the production of this protein, to recombinant cells which contain this expression system, to the human kallikrein so produced, and to a method of producing human kallikrein by culturing these transformed cells.

In the aspect of polymorphism detection, the invention is directed to a method of predicting the likelihood of development of hypertension in an individual, which method comprises detecting the presence or absence of one or more polymorphisms in the renin, ANF, or kallikrein-encoding gene regions. (Polymorphism refers, in the context of the present invention to a location in the DNA of the genome which is different in various individuals. Usually, for a given location in the genome where such polymorphism occurs, a majority of alleles present in a population of individuals will be of one form, and a minority of the alleles present in the population will be of another. For convenience, and for want of a better general term, the form found in the minority of alleles will be referred to as "the polymorphism", using the form present in the majority as a reference. This is in analogy to the manner in which, for example, the term "isotope" is often used to refer to the less common nuclides of an element.)

Specifically, the invention includes:

detecting the presence or absence of a 5 kb or of a 9 kb BglI digestion fragment hybridizing to renin 5' probe, or its substantial equivalent; and/or

detecting the presence of absence of a 0.9 kb digestion fragment hybridizing to a renin 5' or renin 3' probe, or their substantial equivalents; and/or

detecting the presence or absence of a 20 kb or of a 24 kb BglII digestion fragment hybridizing to a renin 5' or renin 3' probe, or their substantial equivalents; and/or

detecting the presence or absence of a 6.2 kb or of a 9.0 kb HindIII digestion fragment hybridizing to renin 3' probe or its substantial equivalent; and/or

detecting the presence or absence of a 3.9 kb EcoRI digestion fragment hybridizing to a mixture of the renin 5' and renin 3' probes or its substantial equivalent; and/or

detecting the presence or absence of a 9.8 kb or of a 11 kb TaqI digestion fragment hybridizing to renin 5' or renin 3' probes, or their substantial equivalents; and/or

detecting the presence or absence of a 1.4 kb or of a 1.8 kb BanII digestion fragment hybridizing to a mixture of the "3 oligo" probes, or their substantial equivalents; and/or

detecting the presence or absence of a 4.1 kb or of a 6.2 kb BglI digestion fragment hybridizing to a mixture of the "3 oligo" probes, or their substantial equivalents; and/or

detecting the presence or absence of a 9.1 kb or of a 6.5 kb BglII digestion fragment hybridizing to a mixture of the "3 oligo" probes, or their substantial equivalents; and/or

detecting the presence or absence of a 5.2 kb or of a 11.8 kb EcoRI digestion fragment hybridizing to a mixture of the "3 oligo" probes, or their substantial equivalents; and/or

detecting the presence or absence of a 15 kb EcoRI digestion fragment hybridizing to λHK1 probe or its substantial equivalent; and/or

detecting the presence or absence of a 3.1 kb PstI digestion fragment hybridizing to λHKl probe or its substantial equivalent; and/or

detecting the presence or absence of a 4.5 kb StuI digestion fragment hybridizing to λHKl probe or its substantial equivalent.

In still another aspect, the invention relates to determination of a genetic fingerprint of a subject, which may be useful in general, such as in the determination of parentage, or which relates generally to cardiovascular disorders, using polymorphisms of the renin, kallikrein, and/or ANF gene region. The invention is also directed to kits suitable for performing the methods of the invention.

Brief Description of the Drawings

Figure 1 shows the DNA sequence of the renin 5' and renin 3' probes.

Figure 2 shows the DNA sequence of the ANF "3-oligo" probes.

Figure 3 shows the DNA sequence of the λHKl probe.

Figure 4 shows an autoradiogram performed using BglI-digested DNAs from 15 individuals, which digests have been probed by renin 5' cDNA.

Modes of Carrying Out the Invention

1.  Detection of Polymorphisms

In the description below, distances of polymorphisms from reference points and lengths of deletions are given, when known, in bp or kb. Where the sequence is known, such measures can be quite precise, but when assessed by measuring fragment sizes on gels or by other experimental means, these measures contain a margin of uncertainty, as is well understood in the art. In general, for measures of >4 kb, the margin of uncertainty is $\pm$ ~ 0.3 kb; for smaller lengths, the error is $\pm$ ~ 10%.

A. Techniques for Detection of Polymorphisms

Application of the method of the invention to predict potential hypertensive individuals or to obtain a genetic "fingerprint" based on some or all of the polymorphisms associated with the general genomic renin, kallikrein, and ANF regions, employs standard techniques of DNA extraction, purification, restriction enzyme digestion, and size separation. Techniques for hybridization with probe and detecting successfully hybridized substrate arranged according to molecular weight are also well known to those in the art. The general approach to finding and detecting the significant polymorphisms is the following:

DNA is extracted from the somatic cells of the individual to be tested, for example from leukocytes, placental cells, cultured fibroblasts, or, in the case of fetal individuals, from cells of the amniotic fluid. In ordinary circumstances, the test material comprises leukocytes from a blood sample -- 25-50 ml blood yields 200-400 μg DNA. The high molecular weight DNA fraction is separated, and subjected to treatment with a particular, selected restriction enzyme, such as, for example, EcoRI, BamHI, MstI, XmnI, and the like. After digestion of the high molecular weight DNA, the digest is applied to a polyacrylamide or agarose gel and subjected to electrophoresis to obtain separation of the DNA fragments resulting from restriction enzyme digestion into positions on the gel determined by the size (length) of the fragment. The contents of the gel are then replicated by transferring to a nitrocellulose filter or other suitable matrix for use as a probe hybridization support. The DNA fragments, either before or after transfer to the nitrocellulose filter replica, are treated with a denaturant such as sodium hydroxide/salt. The denatured, single-stranded DNA, replicated electrophoresis patterns are probed with labeled (usually by $^{32}$P) single-stranded DNA fragments. Other labels besides radioactivity, such as fluorescent molecules may also be used.

Depending on the probe selected, fragments will be detected which derive from a particular region on the genome. For example, in the method of the invention, a cDNA sequence from the renin, kallikrein, or ANF gene sequences is used as a probe. Therefore, the only fragments which will appear on the hybridized filters are those which contain sequences complementary to this probe--i.e., only those which have not been severed either in the genome itself or by the restriction enzyme cleavage from the complementary renin, kallikrein, or ANF fragment. Stated in another way, by using a particular probe, alterations in the genome which are proximal to sequences corresponding to that probe are detected.

The specific procedures used in the general process described in the preceding paragraphs are understood in the art. Procedures for DNA extraction from somatic cells may, for example, be found in Kan, Y.W., et al, Proc Natl Acad Sci (USA) (1978) 75:5631-5635; Taylor, J.M., et al, Nature (1984) 251:392-393; and Kan, Y.W., et al, N Eng J Med (1977) 297:1080-1084. Further improvements which permit rapid extraction of the DNA are also disclosed by Law, D. G.,

et al. Gene (1984) 28:153-158. Techniques for size separation of the restriction enzyme treated DNA fragments are also described in the foregoing references. Restriction enzyme digestions are generally standard in the art and are carried out under buffer, ionic strength, and temperature conditions which are specified by the manufacturer of the particular restriction enzyme.

Transfer to nitrocellulose or other support and probing by prehybridization with nonspecific DNA followed by hybridization with labeled probe are also standard procedures disclosed, for example, in the foregoing references and by Southern, E., (supra). The section of the genome which is fingerprinted or otherwise subject to study using the results is, of course, dependent on the nature of the probe. The probes useful in the present invention are selected from the renin, kallikrein, or ANF gene.

B.  Nature of the Gene Regions and Probes Useful in the Invention

The fragment pattern obtained is diagnostic for a particular polymorphism if the probe selected is complementary to a DNA sequence sufficiently proximal to the polymorphism on the genome that it is not severed from the polymorphism by the restriction cleavage, and has a low probability of being segregated from the polymorphism by crossing over. Acceptable distance limits between the region of probe complementarity and the polymorphism are therefore arbitrary. Generally, probes which hybridize to DNA sequences within 10 kb upstream or downstream of the polymorphism give acceptable results. Occasionally, the pattern of restriction enzyme cleavage may place a distal probe hybridization site on a fragment irrelevant to the polymorphism. The closer the probe to the polymorphism, the greater the range of usable restriction enzymes.

Accordingly, as used herein, a probe which is a "substantial equivalent" to a specified probe is one which hybridizes to the same fragment length in digests of DNA from individuals with a particular polymorphism when the same restriction enzyme is used. Thus, for the "BglII" polymorphism of the renin gene, when BglII digestion is used for diagnosis, the renin 5' and renin 3' probes (described below) are equivalent. For HindIII digestion, they are not. (Of course, the designated probe can be modified in a trivial manner by being made longer or shorter or by selecting a slightly displaced sequence, and the modified probe will be a substantial equivalent.)

cDNA probes are conveniently labeled by nick translation using $\alpha$ [$^{32}$P] dCTP and $\alpha$ [$^{32}$P] dGTP. Oligomer probes are conveniently labeled by kinasing. However, other means to label probes using, for example, fluorescent label or other detectable moiety could also be used. In addition it may be possible to detect probe hybridized to the genetic fragments using other means, such as by reaction with antibody specific for double-stranded DNA. cDNA probes which are complementary only to the exon regions of the gene and which span over intron regions are workable in the method of the invention.

Probes which hybridize to the renin, kallikrein, and ANF regions of the genome were selected because of the known relationship between the products of these genes and blood pressure.

Renin is a critical protein in the angiotensin system, which controls vasoconstriction/dilation. In this system, the vasoactive agent is angiotensin-II, an octapeptide which acts directly to constrict vascular smooth muscle and to induce the release aldosterone from the adrenal cortex. The level of angiotensin-II is determined by the balance between its rate of inactivation by angiotensinases and its rate of formation in a two step process from angiotensinogen. The rate-limiting step in the conversion of angiotensinogen to angiotensin-II is the step catalyzed by renin -- the conversion of angiotensinogen to angiotensin-I. The second step is mediated by a "converting enzyme". Renin is secreted in vivo by the juxtaglomerular cells of the kidney, and is synthesized as a prorenin precursor, which is then processed to give the active renin form. The involvement of renin in the regulation of blood pressure has been established by the utility of inhibitors of renin secretion or direct renin inhibitors. See, for example, Haber, E., Hypertension and the Angiotensin System: Therapeutic Approaches (1984) Raven Press, 133-145; Gagnol, J.P., et al, Abstracts International Society of Hypertension (1984) 10:376.

The ANF gene is also considered a relevant portion of the genome in controlling hypertension since the atrial natriuretic factor produced by this gene controls the excretion of salt from the system. The relationship between high salt and high blood pressure is well established. Therefore, factors which control the level of sodium ion in the system are relevant also to the control of blood pressure.

The kallikrein region is relevant, since the product of this gene is a protein catalyzing the conversion of kininogen to kinin, a known vasodilator. Indeed, kallikrein has been used by injection in the treatment of cardiovacular and cerebrovascular disease, and the urinary excretion of kallikrein is reduced in essential hypertension. Review of the influence of the kallikrein/kinin system on blood pressure are found in Lavinsky, N.G., *Circ Res* (1979) **44**: 441-451; and Carretero, O.A., et al, *M J Physiol* (1980) **238**: F247-F255.

## C. Polymorphisms Relevant to Hypertension

### C.1 Polymorphisms of the Renin Gene Region

Two probes have been used to detect polymorphisms in the region of the renin gene, the renin 5' and the renin 3' probe. Of course, other equivalent probes can be used, including simple modifications of the exemplified probes, which are constructed by shortening or lengthening the probe sequences or by making minor alterations in the base sequence which do not significantly affect the hybridizing power of the probe. In addition, probes which represent DNA sequences which are slight displacements of the exemplified probes in the genome are also usable.

The renin 5' probe represents the codons for the first 198 amino acids of the mature renin sequence, and includes the codons for the amino acids of the pre-sequence. The renin 3' probe contains the remaining mature protein codons. It will be recalled that the labeling process fragments the probe, and thus the presence of intron regions in the target gene does not undermine the effectiveness of the probes.

Five polymorphisms of the renin region have been identified. These are designated BglI, BglII, RsaI, HindIII, and TaqI below. Correlations of such polymorphisms with hypertension are analyzed as follows:

The findings are interpreted in terms of the relative risk of persons having the polymorphism to show the disease, compared to those having an absence of the polymorphism. These "relative incidence" values are calculated according to Wolf, B., _Ann Hum Genet_ (1955) 19:251. As applied to the assays for propensity for hypertension, the relative incidence is calculated as equal to:

$$\frac{PP \times CN}{PN \times CP}$$

where

PP is the number of hypertensive patients having the polymorphism;

PN is the number of hypertensive patients not having the polymorphism;

CP is the number of controls having the polymorphism;

CN is the number of controls not having the polymorphism.

The value calculated by this ratio, if significantly greater than 1, indicates that the persons having the polymorphism are at a greater risk of having hypertension; a value less than 1 shows protection against hypertension.

### C.2 Polymorphisms of the ANF Region

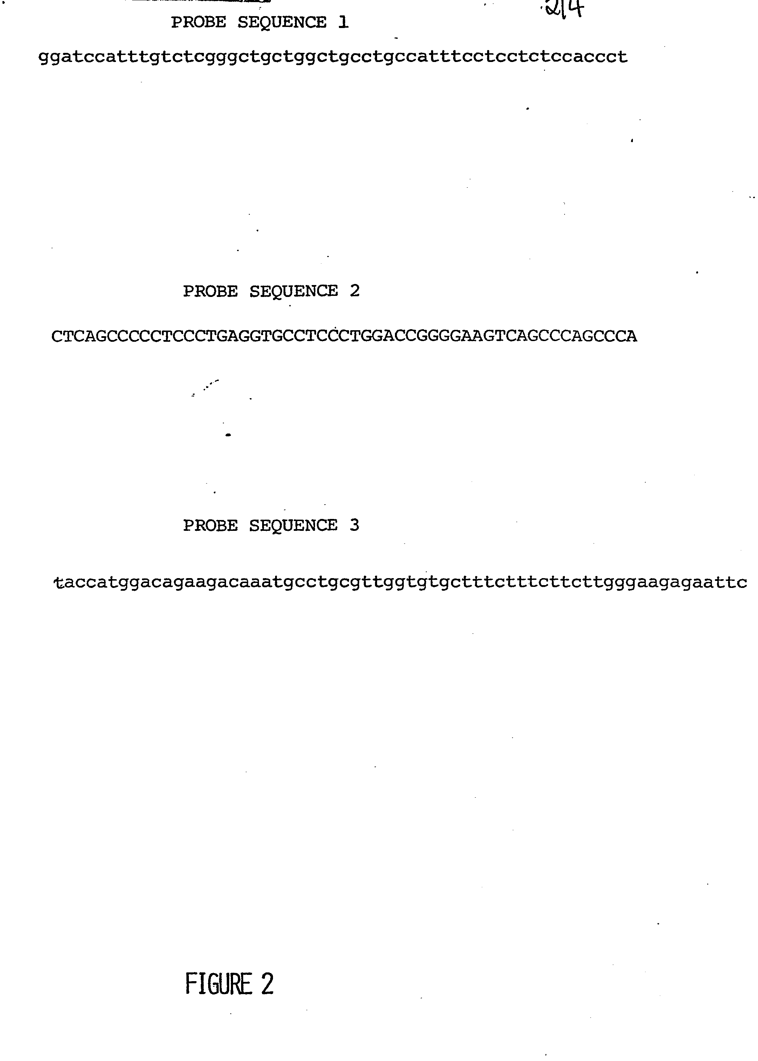

Four polymorphisms have been found in the ANF region, using a mixture of 3 oligomeric probes corresponding to three regions of the ANF gene, as shown in Figure 2. They are designated BanII, BglI, BglII, and EcoRI below. Correlation to hypertension is established as set forth in ¶C.1 above.

0234095

### C.3 Polymorphisms of the Kallikrein Region

A cDNA probe containing the entire coding sequence for human kallikrein, designated λHK1, was used to probe the kallikrein region. Three polymorphisms of the kallikrein region, designated EcoRI, PstI, and StuI, have been identified as set forth in the examples below, and their correlation to hypertension is established as set forth in ¶C.1 above.

### D. Kits

The reagents suitable for applying the method of the invention to detect the appropriate polymorphisms may be packaged into convenient kits providing the necessary materials, packaged into suitable containers, and, optionally, suitable containers or supports useful in performing the assay. The essential components of the assay include the restriction enzyme associated with the polymorphism, and a suitable probe. Additionally, packages containing concentrated forms of reagents used for hybridization, prehybridization, DNA extraction, etc. may be included if desired. In particular, however, labeled probe, or reagents suitable to form conveniently labeled probe, are useful in facilitating the conduct of the method of the invention.

### 2. Cloning and Expression of the Kallikrein Probe

### A. Description of General Kallikrein-Related Parameters

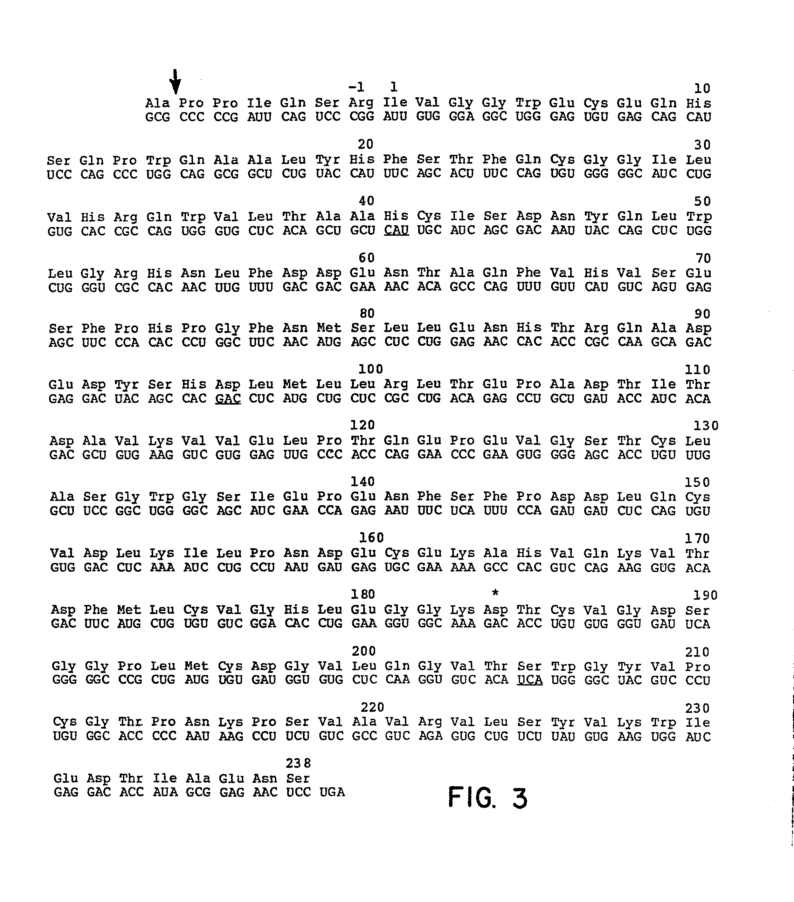

As used herein, human kallikrein refers to a protein which exhibits the biological activity spectrum of kallikrein isolated from human urine, and, specifically, is capable of cleaving specific substrate kininogen to kinin. The human kallikrein of the amino acid sequence shown herein in Figure 3 is exemplified; however it is understood that modifications of this sequence may be made either accidentally or deliberately in order to provide additional favorable properties, which do not destroy the fundamental biological activity of the protein. Such modifications are also intended to be included in the definition, as are conventional modifications of protein structures associated with post-translational processing and environmental factors such as pH.

It is also understood that the term "recombinant host cells" as used herein includes not only the specific recombinant host cells transformed with a particular DNA sequence or vector, but also cultures thereof and progeny thereof. It is also well known that such progeny may not be entirely identical with their parents due to chance mutation or other environmental factors. However, these progeny are included so long as their characteristic property of ability to express the gene for human kallikrein is retained.

The kallikrein produced by recombinant host cells as set forth herein is useful as a vasodilating agent in the treatment of a number of indications for which the material isolated from human urine has been used, including cardioateriosclerosis, hypertension, coronary cardiopathy, exterminal peripheral hemokinetic disorder, Nenilre's syndrome and retinal telangrectasis. Human kallikrein may also substitute for tissue plasminogen activator in the dissolution of blood clots as it is known to convert plasminogen to plasmin.

Formulations suitable for administration of kallikrein and dosage ranges for this pharmaceutical are well known in the art.

The DNA sequence encoding human kallikrein is useful, as shown herein, in the detection of genetic polymer plasma. It may be also expressed in a variety of expression systems as set forth below. The DNA sequence set forth in Figure 3 may be directly ligated into expression vectors, as exemplified for production of the protein intracellu- larly. Alternatively, the DNA sequence shown may be ligated to a leader sequence operable in the specific host cell to produce a secreted protein. Leader DNA seuqences operable in mammalian host cells are, for example, known in the art. The DNA encoding amino acids -26 - (-6) of the human pregrowth hormone sequence may, for example, be used (Martial, J.A., et al. Science (1979) 205:602-607); or that associated with the rat sub-maxillary gland secretion of kallikrein (Ashley, P.L., et al. Biochemistry (1985) 24:4512-4520).

## B. Hosts and Control Sequences

Both procaryotic and eucaryotic systems may be used to express the human kallikrein encoding sequences; procaryotic hosts are, of course, the most convenient for cloning procedures. Procaryotes most frequently are represented by various strains of E. coli; however, other microbial strains may also be used. Plasmid vectors which contain replication sites, selectable markers and control sequences derived from a species compatible with the host are used; for example, E. coli is typically transformed using derivatives of pBR322, a plasmid derived from an E. coli species by Bolivar, et al. Gene (1977) 2:95. pBR322 contains genes for ampicillin and tetracycline resistance, and thus provides multiple selectable markers which can be either retained or destroyed in constructing the desired vector. Commonly used procaryotic control sequences which are defined herein to include promoters for transcription initiation, optionally with an operator, along with ribosome binding site sequences, include such commonly used promoters as the beta-lactamase (penicillinase) and lactose (lac) promoter systems (Chang, et al, Nature (1977) 198:1056) and the tryptophan (trp) promoter system (Goeddel, et al Nucleic Acids Res (1980) 8:4057) and the lambda derived $P_L$ promoter and N-gene ribosome binding site (Shimatake, et al, Nature (1981) 292:128).

In addition to bacteria, eucaryotic microbes, such as yeast, may also be used as hosts. Laboratory strains of Saccharomyces cerevisiae, Baker's yeast, are most used although a number of other strains or species are commonly available. Vectors employing, for example, the 2 μ origin of replication of Broach, J. R., Meth Enz (1983) 101:307, or other yeast compatible origins of replication (see, for example, Stinchcomb, et al, Nature (1979) 282:39, Tschumper, G., et al, Gene (1980) 10:157 and Clarke, L, et al, Meth Enz (1983) 101:300) may be used. Control sequences for yeast vectors include promoters for the synthesis of glycolytic enzymes (Hess, et al, J Adv Enzyme Reg (1968) 7:149; Holland, et al, Biochemistry (1978) 17:4900). Additional promoters known in the art include the promoter for 3-phosphoglycerate kinase (Hitzeman, et al, J Biol Chem (1980) 255:2073). Other promoters, which have the additional advantage of transcription controlled by growth conditions and/or genetic background are the promoter regions for alcohol dehydrogenase 2, isocytochrome C, acid phosphatase, degradative enzymes associated with nitrogen metabolism, the alpha factor system and enzymes responsible for maltose and galactose utilization.   It is also believed terminator sequences are desirable at the 3' end of the coding sequences. Such terminators are found in the 3' untranslated region following the coding sequences in yeast-derived genes.

It is also, of course, possible to express genes encoding polypeptides in eucaryotic host cell cultures derived from multicellular organisms.  See, for example, Axel, et al, 4,399,216.  These systems have the additional advantage of the ability to splice out introns and thus can be used directly to express genomic fragments.  Useful host cell lines include VERO and HeLa cells, and Chinese hamster ovary (CHO) cells. Expression vectors for such cells ordinarily include promoters and control sequences compatible with mammalian cells such as, for example, the commonly used early and late promoters from Simian Virus 40 (SV 40) (Fiers, et al, Nature (1978) 273:113), or other viral promoters such as those derived from polyoma, Adenovirus 2, bovine papilloma virus, or avian sarcoma viruses. The controllable promoter, hMTII (Karin, M., et al, Nature (1982) 299:797-802) may also be used.  General aspects of mammalian cell host system transformations have been described by Axel (supra).  It now appears, also that "enhancer" regions are important in optimizing expression: these are, generally, sequences found upstream or downstream of the promoter region in non-coding DNA regions.  Origins of replication may be obtained, if needed, from viral sources.  However, integration into the chromosome is a common mechanism for DNA replication in eucaryotes.

C. Transformation and Recombinant DNA Vector
   Constructions

Techniques for these manipulations are standard and well known in the art. For transformation the calcium treatment employing calcium chloride, as described by Cohen, S.N., Proc Natl Acad Sci (USA) (1972) 69:2110, or the RbCl$_2$ method described in Maniatis, et al, Molecular Cloning: A Laboratory Manual (1982) Cold Spring Harbor Press, p. 254 and Hanahan, D., J Mol Biol (1983) 166:557-580 may be used for procaryotes or other cells which contain substantial cell wall barriers. For mammalian cells without such cell walls, the calcium phosphate precipitation method of Graham and van der Eb, Virology (1978) 52:546, optionally as modified by Wigler, M., et al, Cell (1979) 16:777-785 may be used. Transformations into yeast may be carried out according to the method of Beggs, J.D., Nature (1978) 275:104-109 or of Hinnen, A., et al, Proc Natl Acad Sci (USA) (1978) 75:1929.

Construction of suitable vectors containing the desired coding and control sequences employs standard ligation and restriction techniques which are well understood in the art. Isolated plasmids, DNA sequences, or synthesized oligonucleotides are cleaved, tailored, and religated in the form desired.

The DNA sequences which form the vectors are available from a number of sources. Backbone vectors and control systems are generally found on available "host" vectors which are used for the bulk of the sequences in construction. Typical sequences have been set forth above. For the pertinent coding sequence, initial construction may be, and usually is, a matter of retrieving the appropriate sequences from cDNA or genomic DNA libraries. However, once the sequence is disclosed it is possible to synthesize the entire gene sequence in vitro starting from the individual nucleoside derivatives. The entire gene sequence for genes of sizeable length, e.g., 500-1000 bp may be prepared by synthesizing individual overlapping complementary oligonucleotides and filling in single stranded nonoverlapping portions using DNA polymerase in the presence of the deoxyribonucleotide triphosphates. This approach has been used successfully in the construction of several genes of known sequence. See, for example, Edge, M. D., Nature (1981) 292:756; Nambair, K. P., et al. Science (1984) 223:1299; Jay, Ernest. J. Biol Chem (1984) 259:6311

Synthetic oligonucleotides are prepared by either the phosphotriester method as described by Edge, et al. Nature (supra) and Duckworth, et al. Nucleic Acids Res (1981) 9:1691 or the phosphoramidite method as described by Beaucage, S.L., and Caruthers, M.H., Tet Letts (1981) 22:1859 and Matteucci, M.D., and Caruthers, M.H., J Am Chem Soc (1981) 103:3185 and can be prepared using commercially available automated oligonucleotide synthesizers. Kinasing of single strands prior to annealing or for labeling is achieved using an excess, e.g., approximately 10 units of polynucleotide kinase to 1 nmole substrate in the presence of 50 mM Tris, pH 7.6, 10 mM $MgCl_2$, 5 mM dithiothreitol, 1-2 mM ATP, 1.7 pmoles $\gamma$32P-ATP (2.9 mCi/mmole), 0.1 mM spermidine, 0.1 mM EDTA.

Once the components of the desired vectors are thus available, they can be excised and ligated using standard restriction and ligation procedures.

Site specific DNA cleavage is performed by treating with the suitable restriction enzyme (or enzymes) under conditions which are generally understood in the art, and the particulars of which are specified by the manufacturer of these commercially available restriction enzymes. See, e.g., New England Biolabs, Product Catalog. In general, about 1 µg of plasmid or DNA sequence is cleaved by one unit of enzyme in about 20 µl of buffer solution; in the examples herein, typically, an excess of restriction enzyme is used to insure complete digestion of the DNA substrate. Incubation times of about one hour to two hours at about 37°C are workable, although variations can be tolerated. After each incubation, protein is removed by extraction with phenol/chloroform, and may be followed by ether extraction, and the nucleic acid recovered from aqueous fractions by precipitation with ethanol. If desired, size separation of the cleaved fragments may be performed by polyacrylamide gel or agarose gel electrophoresis using standard techniques. A general description of size separations is found in Methods in Enzymology (1980) 65:499-560.

Restriction cleaved fragments may be blunt ended by treating with the large fragment of E. coli DNA polymerase I (Klenow) in the presence of the four deoxynucleotide triphosphates (dNTPs) using incubation times of about 15 to 25 min at 20 to 25°C in 50 mM Tris pH 7.6, 50 mM NaCl, 6 mM $MgCl_2$, 6 mM DTT and 0.1-1.0 mM dNTPs. The Klenow fragment fills in at 5' single-stranded overhangs but chews back protruding 3' single strands, even though the four dNTPs are present. If desired, selective repair can be performed by supplying only one of the, or selected, dNTPs within the limitations dictated by the nature of the overhang. After treatment with Klenow, the mixture is extracted with phenol/chloroform and ethanol precipitated. Treatment under appropriate conditions with S1 nuclease

or BAL-31 results in hydrolysis of any single-stranded portion.

Ligations are performed in 15-50 µl volumes under the following standard conditions and temperatures: for example, 20 mM Tris-Cl pH 7.5, 10 mM MgCl$_2$, 10 mM DTT, 33 µg/ml BSA, 10 mM-50 mM NaCl, and either 40 µM ATP, 0.01-0.02 (Weiss) units T4 DNA ligase at 0°C (for "sticky end" ligation) or 1 mM ATP, 0.3-0.6 (Weiss) units T4 DNA ligase at 14°C (for "blunt end" ligation). Intermolecular "sticky end" ligations are usually performed at 33-100 µg/ml total DNA concentrations (5-100 nM total end concentration). Intermolecular blunt end ligations are performed at 1 µM total ends concentration.

In vector construction employing "vector fragments", the vector fragment is commonly treated with bacterial alkaline phosphatase (BAP) or calf intestinal alkaline phosphatase (CIP) in order to remove the 5' phosphate and prevent selfligation of the vector. Digestions are conducted at pH 8 in approximately 10 mM Tris-HCl, 1 mM EDTA using about 1 unit of BAP or CIP per µg of vector at 60° for about one hour. In order to recover the nucleic acid fragments, the preparation is extracted with phenol/chloroform and ethanol precipitated. Alternatively, religation can be prevented in vectors which have been double digested by additional restriction enzyme digestion and separation of the unwanted fragments.

For portions of vectors derived from cDNA or genomic DNA which require sequence modifications, site specific primer directed mutagenesis may be used (Zoller, M.J., and Smith, M. Nucleic Acids Res (1982) 10:6487-6500 and Adelman, J.P., et al, DNA (1983) 2:183-193). This is conducted using a primer synthetic oligonucleotide complementary to a single stranded phage DNA to be mutagenized except for limited mismatching, representing the desired mutation. Briefly, the synthetic oligonucleotide is used as a primer to direct synthesis of a strand complementary to the phage, and the resulting partially or fully double-stranded DNA is transformed into a phage-supporting host bacterium. Cultures of the transformed bacteria are plated in top agar, permitting plaque formation from single cells which harbor the phage.

Theoretically, 50% of the new plaques will contain the phage having, as a single strand, the mutated form; 50% will have the original sequence. The resulting plaques are washed after hybridization with kinased synthetic primer at a wash temperature which permits binding of an exact match, but at which the mismatches with the original strand are sufficient to prevent binding. Plaques which hybridize with the probe are then picked, cultured, and the DNA recovered.

Correct ligations for plasmid construction are confirmed by first transforming E. coli strain MC1061 obtained from Dr. M. Casadaban (Casadaban, M., et al, J Mol Biol (1980) 138:179-207) or other suitable host with the ligation mixture. Successful transformants are selected by ampicillin, tetracycline or other antibiotic resistance or using other markers depending on the mode of plasmid construction, as is understood in the art. Plasmids from the transformants are then prepared according to the method of Clewell, D.B., et al, Proc Natl Acad Sci (USA) (1969) 62:1159, optionally following chloramphenicol amplification (Clewell, D.B., J Bacteriol (1972) 110:667). Several mini DNA preps are commonly used, e.g., Holmes, D.S., et al, Anal Biochem (1981) 114:193-197 and Birnboim, H.C., et al, Nucleic Acids Res (1979) 7:1513-1523. The isolated DNA is analyzed by restriction and/or sequenced by the dideoxy nucleotide method of Sanger, F., et al. Proc Natl Acad Sci (USA) (1977) 74:5463 as further described by Messing, et al, Nucleic Acids Res (1981) 9:309, or by the method of Maxam, et al, Methods in Enzymology (1980) 65:499.

Examples

## Example 1
### Detection of Polymorphisms

The following example is specific with respect to the probes exemplified and with respect to the precise conditions of DNA extraction, probe hybridization, etc. It is understood that these factors are illustrative but not limiting. The essential features of the invention as it relates to detection of a particular polymorphism are (1) the selection of enzyme, and (2) the selection of probe. For a particular restriction enzyme, a number of substantially equivalent probes which are not segregated from the identified fragment by this restrictions cleavage are usable. Alternatively, other restriction enzymes may be used in conjunction with a probe which hybridizes in particularly close proximity to the polymorphism.

In the above sense, the particular restriction enzyme and cDNA probe chosen are arbitrary. However, it should be noted, as is understood in the art, that the efficacy of the probe is enhanced as it moves closer to the site of the polymorphism. Otherwise, additional cleavage points may be encountered between the polymorphism and the probe, and also the probing site may be separated from the site of the polymorphism during replication by crossing-over events.

1. Procedures for Analysis

Leukocytes were obtained from freshly drawn blood collected from each of the human subjects, and high molecular weight genomic DNA was isolated by the procedure of Law, D. J., et al, Gene (1984) 28:153-158.

High molecular weight DNA was divided into portions and each was digested to completion with one of the various restriction enzymes under conditions recommended by the suppliers (New England Biolabs and Bethesda Research Laboratories). The digests were electrophoresed in horizontal agarose gels in 30 mM $NaH_2PO_4$, 36 mM Tris, 1 mM EDTA, pH 7.7. After electrophoresis, DNA fragments were denatured in situ in 0.5 M NaOH/1.5 M NaCl for 2 x 10 min, neutralized in 1 M ammonium acetate pH 7.2 for 2 x 10 min, and transferred overnight onto nitrocellulose paper (Schleicher and Schuell). The filters were rinsed in 2 x SSC (1x SSC is 0.15 M NaCl, 0.015 M sodium citrate pH, 7.4) and baked for 2 hr at 80°C in vacuo and then were prehybridized for 5 hr in plastic bags using 0.3 ml/cm$^2$ of a solution containing 5x SSPE (1x SSPE is 10 mM Na phosphate pH 7.4, 0.18 M NaCl and 1 mM EDTA) containing 5x Denhardt's solution (1 x Denhardt's contains 0.2 mg/ml each of Ficoll, polyvinylpyrrolidone and bovine serum albumin), 40% (vol/vol) formamide, and 250 µg/ml sheared and denatured salmon sperm DNA, and hybridized overnight in the same bag in 0.1 ml/cm$^2$ of 5x SSPE, 1x Denhardt's solution, 40% (vol/vol) formamide, 10% dextran sulfate, and 100 µg/ml sheared and denatured salmon sperm DNA, mixed with 100 ng per bag (containing 1 or 2 filters) of the appropriate $^{32}$P-labeled probe, as discussed below. Prehybridization and hybridization were performed at 42°C.

Filters were then washed twice at room temperature in 2x SSC and twice at 65°C in 2x SSC, 1x Denhardt's solution. DNA sequences hybridized to the $^{32}$P-labeled probes were visualized by autoradiography using XAR-5 films (Kodak) and Cronex intensifying screens (Dupont) at -70°C for 18 hr to 2 days.

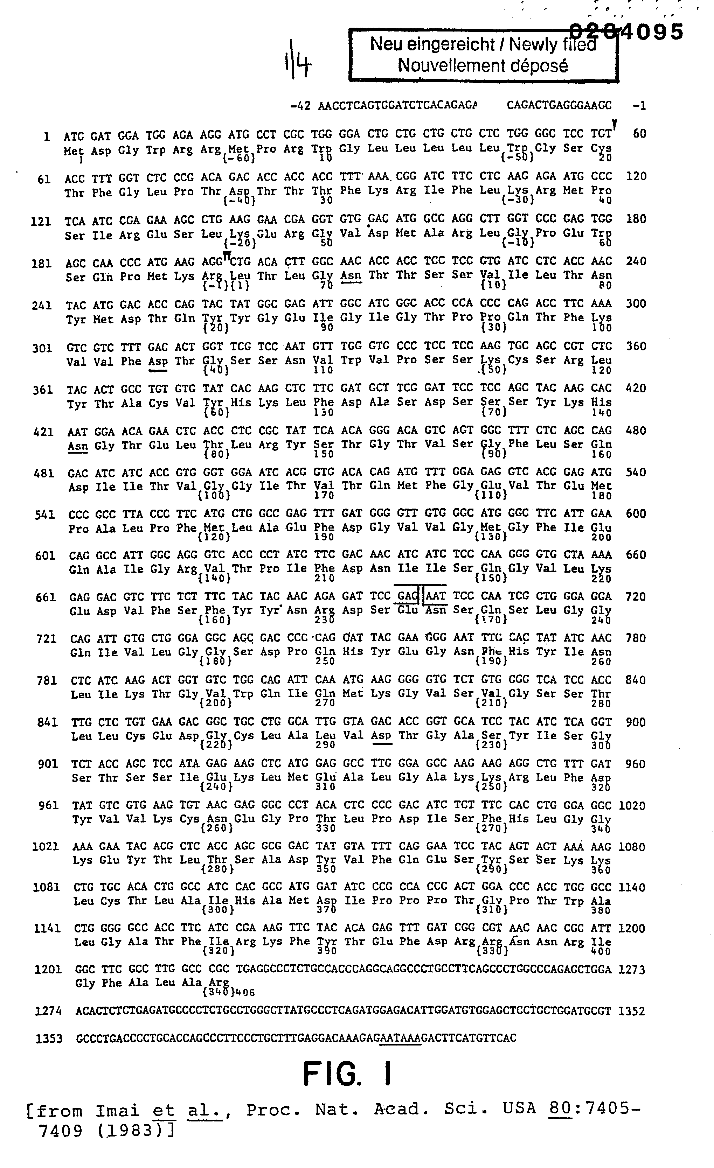

Two specific probes are used for the renin region in the illustrations below: renin 5' probe and renin 3' probe, as shown in Figure 1.

A mixture of three oligomeric probes was used for the ANF region: a 53-mer corresponding to a sequence at the 5' end, a 50-mer corresponding to a sequence at the middle of the gene and a 63-mer corresponding to a sequence at the 3' end. These oligomers are shown in Figure 2.

A probe designated λHK1 was used for the kallikrein region. The sequence of this probe is shown in Figure 3.

The methods used to prepare these probes are described; however, the complete DNA sequences are given so that synthetic methods may also be used (see above).

For use in hybridization, the probes are labeled to a specific activity of 2.0-5.0 x $10^8$ cpm/µg by nick-translation, using the BRL nick-translation kit (Bethesda Research Laboratories) under recommended conditions with α [$^{32}$P] dCTP (800 Ci/mM; Amersham Corporation) in the presence of unlabeled dATP and dTTP. The probes were denatured just before the hybridization step by incubation for 5 min in a boiling water bath, followed by rapid cooling in ice water.

To prepare the renin probes, a cDNA library was prepared from oligo-dT-primed polyA$^+$ RNA, purified from the kidney of a human accident victim. The cDNA library was constructed in the bacteriophage vector λgt10 and probed with appropriate fragments of Charon-4 human renin genomic clones which had been obtained from a human genomic library by probing with mouse submaxillary gland renin cDNA fragments. The λgt10 library was prepared by standard procedures which include obtaining double-stranded cDNA from the mRNA, blunt-ending the cDNA with DNA polymeraseI (Klenow), adding commercially available EcoRI linkers, cleaving with EcoRI, and ligating the fragments into EcoRI-digested λgt10 vectors. Two positively responding cDNA clones together, when sequenced using the dideoxy method of Sanger (supra), were shown to contain the entire renin-encoding sequence and 3' untranslated region except for a missing 7 base pairs at the 5' end of the signal sequence. The two clones include 1211 bp of coding region, followed by the entire 3' 198 bp untranslated region, followed, in turn, by a polyA tail. The assignment of the sequence to the appropriate preprorenin codons and reading frame was made by comparison to the published Imai et al (supra) sequence. Two cloned fragments result because of the unique EcoRI site at position 762, as shown in Figure 1.

The cDNA sequences encoding the preprorenin protein were transferred into pUC9 as the two EcoRI inserts to obtain the cloned vectors pHR1 containing renin 5' probe and pHR2 containing renin '3 probe. pHR1 and pHR2 were prepared by excising the cDNA from λgt10 using EcoRI and ligating each of the resulting fragments into EcoRI-cleaved pUC9. For use as probes, the inserts were removed with EcoRI and labeled.

The ANF probes were prepared from the 2.6 kb genomic clone which encompasses the entire human prepro-ANF gene, as described in Greenberg, G.D., et al.

<u>Nature</u> (1984) <u>312</u>:656-658. This clone has been shown to contain Alu family repetitive sequences as shown in Figure 2. The sequences corresponding to the three probes is shown in the figure - each oligomer corresponds to a different portion of the gene. These probes can be used singly or as a mixture.

The kallikrein probe was prepared from a human kidney cDNA library constructed in λgt10 in a manner precisely analogous to that set forth above. This library was probed with P-32-labeled mouse cDNA clone pMK1, described by Mason et al, <u>Nature</u> (1983) <u>303</u>: 300-307, under conditions of reduced stringency (5 x SSC, 50°C). Twenty positively hybridizing clones were isolated after screening approximately 200,000 recombinant phage. All of the positively hybridizing phage were rescreened by hybridization with a 52-mer oligonucleotide which was based on the amino terminal sequence of human urinary kallikrein (Lottspeich et al, <u>Hoppe Seyler's Z Physio Chem</u> (1979) <u>360</u>: 1947-1950) using codon choices from previously sequenced human genes. A clone designated λHK1 containing the largest (approximately 1 kb) insert was selected and subcloned into M13mp8 for DNA sequencing. The relevant DNA sequence of this clone is shown in Figure 3. This 1 kb insert was subconed into pUC8 for amplification, and removed by digestion with EcoRI for denaturation and use as a probe.

## 2. Polymorphisms of the Renin Gene

Using the renin 5' and renin 3' probes, 68 individuals were tested for polymorphism by the procedure described above. The following polymorphisms were found, as shown in Table 1.

## Table 1

| Poly-morphism | Probe | Enzyme | More Common Fragment | Less Common Fragment | Freq |
|---|---|---|---|---|---|
| BglI | renin 5' | BglI | 5 kb | 9 kb | 0.39 |
| BglII | renin 5' renin 3' | BglII | 20 kb | 24 kb | 0.28 |
| RsaI | renin 5' renin 3' | RsaI | 0.9 kb | – | 0.35 (US) 0.40 (Germany) |
| HindIII | renin 3' | HindIII | 9.0kb | 6.2kb | 0.34 |
| TaqI | renin 5' renin 3' | TaqI | 9.8kb | 11.0kb | 0.22 |
| EcoRI | mixture | EcoRI | – | 3.9kb | 0.025±0.01 |

Frequency in this case as in all others is determined as follows: Each individual has two copies of each chromosome; therefore, at any locus or position on the chromosome each person has two "alleles". If these two alleles are identical, the individual is "homozygous" at this locus; if the two alleles are different, he is heterozygous at this locus. Genetic variation between populations can be quantified using the concept of allele frequency, the proportion of all alleles in a population at the locus that are a particular allele. The frequency of any particular allele in a sample is therefore equal to twice the number of homozygotes for that allele plus the number of heterozygotes for that allele divided by two times the number of individuals in the sample.

For example, in the case of the BglI (5 kb/9 kb) polymorphism there were:

--28 homozygotes of the 5 kb type
--13 homozygotes of the 9 kb type
--27 heterozygotes

The polymorphic allele frequency (9 kb type) is thus:

$$p = \frac{26 + 27}{56 + 26 + 54} \qquad \text{or approximately } 0.39$$

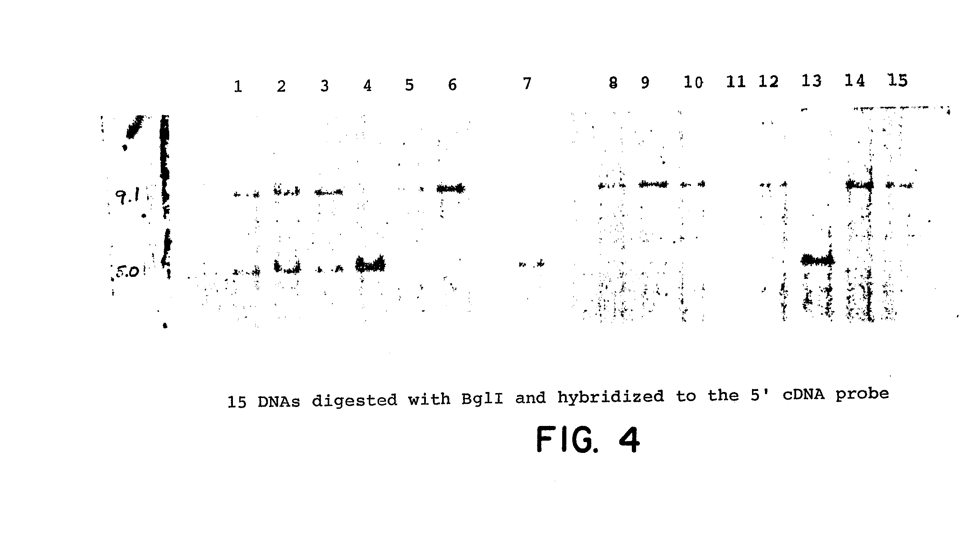

Typical results are shown for the BglI polymorphism in Figure 4. Each lane of the autoradiogram shows the results for one individual probed with renin 5' probe. Lanes 4, 7, 11 and 13 show individuals homozygous for 5 kb fragment, lanes 5, 6, 10, 12, 14, and 15 show individuals homozygous for the 9 kb fragment, and lanes 1-3, 8 and 9 show the results for heterozygous individuals.

3. Polymorphisms of the ANF Gene

There are four known polymorphisms of the ANF gene, as shown in Table 2.

Table 2

| Poly-morphism | Probe | Enzyme | More common Fragment | Less common Fragment | Freq |
|---|---|---|---|---|---|
| BanII | 3 oligo mixture | BanII | 1.8 kb | 1.4 kb | 0.2 |
| BglI | 3 oligo mixture | BglI | 6.2 kb | 4.1 kb | 0.15 |
| BglII | 3 oligo mixture | BglII | 6.5 kb | 9.1 kb | 0.06 |
| EcoRI | 3 oligo mixture | EcoRI | 11.2 kb | 5.2 kb | 0.05 |

The correlation between the presence of the polymorphism above and risk of hypertension is evaluated as set forth above with respect to the polymorphisms of the renin gene.

## 4. Polymorphisms of the Kallikrein Gene

Three polymorphisms were found in the kallikrein gene as shown in Table 3.

### Table 3

| Polymorphism | Probe | Enzyme | Less common Fragment | Freq |
|---|---|---|---|---|
| EcoRI | λHK1 | EcoRI | 15.0 kb | 0.08 |
| PstI | λHK1 | PstI | 3.1 kb | 0.13 |
| StuI | λHK1 | StuI | 4.5 kb | 0.03 |

Correlations with specific symptomologies are obtained as set forth above.

### Example 2

### Cloning and Expression of the Kallikrein Gene

The following description of the isolation of cDNA for human kallikrein and the expression thereof serves to illustrate the invention, but is not intended to limit it. As set forth above, the illustrated sequence can be suitably modified and expression may be obtained in a variety of systems.

### 1. Retrieval of Kallikrein-Encoding cDNA

Messenger RNA was prepared from the kidneys of a young female accident victim by the method of Chirgwin et al, Biochem. (1979) 18:5294-5299. Briefly, 10 g of tissue was homogenized in 50 ml of 5 M guanidine thiocyanate, 0.1 M B-mercaptoethanol, 10 mM Tris, 1 mM EDTA. Extracted RNA was then pelleted through a CsCl cushion and polyA$^+$ RNA was selected by chromatography on oligo(dT)-cellulose (Aviv, et al, Proc Natl Acad Sci (USA) (1972) 80:3015-3019).

A cDNA library was constructed in λgt10 from the total mRNA as described by Huynh, T.V. et al *DNA Cloning Techniques: A Practical Approach* IBL Press, Oxford 1984, D. Glover, ed. Recombinant phage were plated at a density of 50,000 pfu per 37 mm plate on *E. coli* C600 HFL and screened with nick translated probes as described (Maniatis *et al*, "Molec. Cloning, A Lab Manual" (1982) Cold Spring Harbor Press, using $P^{32}$-labeled mouse cDNA clone pMK1 (Mason, et al, *Nature* (1983) 303:300-307) under conditions of reduced stringency (5 X SSC, 50°C).

(Restriction fragments to be used as probe were isolated from agarose gels by electrophoresis onto DE-81 paper (Dretzen, et al, *Anal Biochem* (1981) 112:295-298). Probe DBA was labelled with [α-$^{32}$P] dCTP and [α-$^{32}$P] dATP (Amersham) using a nick translation kit (New England Nuclear) yielding specific activities of $10^7$-$10^8$ cpm/μg.)

Twenty positively hybridizing clones were isolated after screening approximately 200,000 recombinant phage, suggesting that kallikrein mRNA represents some 0.01% of total kidney mRNA. (This result was consistent with subsequent Northen analysis of the same kidney RNA preparation used for generation of the cDNA library.)

All of the positively hybridizing phage were rescreened by hybridization with a 52-mer oligonucleotide 5'-ATCGTGGGCGGCTGGGAATGTGAGCAGCACAGCCACC CCTGGCAGGCTGCTC-3'. This probe was based on the amino-terminal sequence of human urinary kallikrein (Lottspeich, et al, *Hoppe-Seyler's Z Physiol Chem* (1979) 360:1947-1950) using codon choices from previously sequenced human genes. A clone (λHK1) containing the largest (approximately 1,000 bp) insert which hybridized to the amino-terminal probe was selected for further study by subcloning into M13mp8 for direct DNA sequence analysis, as described by Messing et al, Gene (1982) 19:269-276. Chain termination reactions were performed using dideoxynucleotides (P-L Biochemicals), DNA polymerase I (Klenow fragment) and [ $-^{32}$P] dATP (Schreier et al, J Mol Biol (1979) 129:169-172).

The DNA sequence of λHK1 and the amino acid sequence encoded by it is shown in Fig. 3. The coding region is composed of a signal peptide (not complete in λHK-1) followed by a pro-kallikrein of 245 amino acids. Based on homology to the well characterized mouse and rat genes, the pro-peptide consists of 7 residues and mature kallikrein contains 238 amino acids. The characteristic His41, Asp96 and Ser 190 residues which form the active site of serine proteases are all present, as is the Asp184 which determines the ability to cleave after arginine residues (Krieger et al, J. Mol. Biol. (1974) 83:209-230). The amino-terminal 31 amino acids are identical to those published for human urinary kallikrein (Lottspeich, et al, (supra)) confirming the kidney as a site of synthesis for this protein.

## 2. Kallikrein Genes

To investigate the number of kallikrein (or kallikrein-like) genes in the human genome, Southern blots were carried out on restriction digests of DNA isolated from several different individuals as follows:

Restriction digests of human genomic DNA (10 μg) were resolved on 1% agarose (Seakem, ME) in Tris-acetate buffer (40 mM Tris, 10 mM Na acetate, 20 mM NaCl, 2 mM EDTA, pH 8.15), and hybridization was carried out for 12 hours at 50°C in 5 X SSC, 5X Denhardts, 0.1% Sarkosyl, 200 µg/ml denatured salmon sperm DNA, 1 mM EDTA. Filters were washed in several changes of 1 X SSC, 0.1% SDS at 50°C then exposed to X-ray film (Kodak XS-5) in the presence of an intensifying screen (Dupont Cronex lightening plus) at -70°C.

Several bands hybridized to the $P^{32}$-labeled insert from λHK1, suggesting the presence of several related sequences. However, the number of related genes may be significantly less in human DNA than in the mouse genome, since under similar hybridization conditions the pattern of hybridization is far more extensive in mouse DNA. Even in a small sample number of individual DNAs, several restriction fragment length polymorphisms were observed. For example, digestion with EcoRI demonstrates the presence in some individuals of a 15 kb fragment which represents a polymorphic allele with a frequency of 0.08. Similarly PstI (3.1 kb) and StuI (4.5 kb) polymorphisms are present with frequencies of 0.13 and 0.03 respectively, as set forth above.

3. **Construction of Expression Vectors and Expression of Human Kallikrein**

The cDNA clones encoding human kallikrein are most conveniently used to produce the recombinant proteins in a variety of hosts, as set forth above. However, expression in mammalian systems is favored as the host is capable of post translational processing analogous to that experienced by the natively produced protein, and either cDNA or genomic sequences may be used, as the host is also capable of processing introns.

A full length cDNA or genomic human kallikrein encoding clone is prepared for insertion into a host vector, illustrated by but not limited to those described below. The cloned insert is excised with EcoRI (by partial digestion if the insert itself contains EcoRI sites), or other appropriate enzyme, provided with EcoRI linkers if necessary, and then inserted into the host vector pHS1 as described below.

### Construction of pHS1, the Host Vector

The plasmid pHS1 is suitable for expression of inserted DNA in mammalian hosts. It contains 840 bp of the hMT-II sequence from p84H (Karin, M., et al, *Nature* (1982) $\underline{299}$: 297-802) which spans from the HindIII site at position -765 of the hMT-II gene to the BamHI cleavage site at base + 70. To contruct pHS1, plasmid p84H was digested to completion with BamHI, treated with exonuclease BAL-31 to remove terminal nucleotides, and then digested with HindIII. The desired 840 bp fragment was ligated into pUC8 (Vieira, J., et al, *Gene* (1982) $\underline{19}$: 259-268) which had been opened with HindIII and HincII digestion. The ligation mixture was used to transform $\underline{E.\ coli}$ HB101 to Amp$^R$, and one candidate plasmid, designated pHS1, was isolated and sequenced by dideoxy sequencing. pHS1 contains the hMT-II control sequences upstream of a polylinker containing convenient restriction sites.

### Construction of Expression Vectors

The human kallikrein encoding fragments, prepared as above, are ligated into EcoRI digested pHS1 and the ligation mixture used to transform $\underline{E.\ coli}$ MC1061 to Amp$^R$. Successful transformants are screened by restriction analysis, and a strain containing the desired plasmid, pMT-human kallikrein is further propagated to prepare quantities of plasmid DNA.

## Production of Human Kallikrein by Mammalian Recombinants

Chinese hamster ovary (CHO)-K1 cells are grown on medium composed of a 1:1 mixture of F12 medium and DME medium with 12% fetal calf serum. The competent cells are co-transformed with pMT-human kallikrein and pSV2:NEO (Southern, P., et al, _J Mol Appl Genet_ (1982) **1**: 327-341). pSV2:NEO contains a functional gene conferring resistance to the neomycin analog G418. In the transformation, 500 ng of pSV2-NEO and 5 µg of pMT-human kallikrein are applied to a 16 mm dish of cells in a calcium phosphate-DNA co-precipitate according to the protocol of Wigler, M., et al, _Cell_ (1979) **16**: 777-785, with the inclusion of a two minute "shock" with 15% glycerol after four hours of exposure to the DNA. A day later, the cells are subjected to 1 mg/ml G418 to provide a pool of G418-resistant colonies, which are assayed for human kallikrein production and then cloned out.

Successful transformants, also having a stable inheritance of pMT-human kallikrein, are plated at low density for purification of clonal isolates. Small amounts of these isolates are grown in multi-well plates after exposure to $10^{-4}$ M zinc chloride for convenient assay of human kallikrein production. Human kallikrein determinations are made by standard ELISA or radio-immunoassays against the antisera prepared against the appropriate human kallikrein protein using standard methods. Clonal isolates which produce large amounts of the desired human kallikrein are selected.

The cells, which have been shown to produce human kallikrein under suitable conditions, are seeded at 1/10 confluency in basal medium supplemented with 10% fetal calf serum, incubated overnight, and then induced

0234095

for human kallikrein production by addition of zinc chloride in the concentration range of $1 \times 10$ M to 3 $\times 10^{-4}$ M. Human kallikrein levels rise for 7-10 days, under optimal inducing conditions, $2 \times 10$ M $ZnCl_2$.

If desired, the human kallikrein secreted into the medium can be purified according to the procedures set forth above for the native protein, or by other standard methods known in the art.

## Bacterial Expression of Human Kallikrein

The cDNA sequences encoding human kallikrein, which are uninterrupted by introns, are also expressible in bacterial systems. A convenient host vector for expression is pKT52, which contains the "trc" promoter, followed by an ATG start codon.

pKT52 is obtained by simple manipulation of pKK233-2, described by Amman, E., et al, Gene (1985) 40:183-190. pKK233-2 was digested with EcoRI and PvuII, filled in with dATP and dTTP, and religated to obtain the correct construction pKT52. pKT52 contains the desired trc promoter, a downstream ATG start codon, and downstream NcoI, PstI and HindIII sites.

For construction of expression vectors, the human kallikrein-encoding cDNA is obtained by excising with EcoRI or other appropriate enzyme digestion and isolating and, if necessary, modifying the appropriate fragment. The 3' end is prepared for insertion into pKT52 by cutting downstream of the termination codon at any convenient restriction site and supplying PstI or HindIII linkers. The 5' end is prepared by cutting at a site inside the coding sequence and supplying the missing codons and an NcoI site using a synthetic DNA, or by providing an appropriately located NcoI site by

mutagenesis.  The resulting NcoI/HindIII or NcoI/PstI
fragment is then ligated into NcoI/HindIII-digested
pKT52 or NcoI/PstI digested pKT52 to provide the human
kallikrein-encoding cDNA in reading frame with the ATG
start codon.

For bacterial expression, the resulting
expression vectors are used to transform $\underline{E.\ coli}$ MC1061
or other appropriate host cells to Amp$^R$, and the
transformed cells are then grown on M9 medium containing
1 mM IPTG for 3-5 hr to an O.D. of 0.2-0.5.  (IPTG is a
standard inducer for control sequences regulated by the
lac operator.)  The cells are then harvested, lysed by
sonication or treatment with 5% trichloroacetic acid,
and the cell extracts assayed for the desired human
kallikrein.  Human kallikrein can be purified from the
extracts by methods used for the native protein or by
other procedures known in the art.


## Concluding Remarks

Several of the approximately 10 million
polymorphisms existent in the human genome have been
shown to be useful predictors of individuals at risk for
hypertension.  These polymorphisms are detectable as
fragments of predictable size obtained by digestion of
the genomic DNA of the subject individual with a
specified restriction enzyme and probing with specific
DNA sequences herein described.  The availability of
this tool for early diagnosis of individuals at risk for
hypertension permits the early application of
therapeutic measures to prevent the fatal symptomology
of the disease.

## Claims

1. A method for genetic analysis of individual human subjects in particular, for those having a propensity for disorders related to the cardiovascular system, in particular hypertension, which method comprises detecting the presence or absence of one or more polymorphisms selected from the group consisting of:

the BglI polymorphism of the renin gene;
the BglII polymorphism of the renin gene;
the RsaI polymorphism of the renin gene;
the HindIII polymorphism of the renin gene;
the TaqI polymorphism of the renin gene;
the EcoRI polymorphism of the renin gene;
the BanII polymorphism of the ANF gene;
the BglI polymorphism of the ANF gene;
the BglII polymorphism of the ANF gene;
the EcoRI polymorphism of the ANF gene;
the EcoRI polymorphism of the kallikrein gene;
the PstI polymorphism of the kallikrein gene; and

the StuI polymorphism of the kallikrein gene.

2. The method of claim 1 wherein each modification is detected in total human genomic DNA by, respectively,

digestion with BglI and detection of a 9 kb fragment using renin 5' probe or a substantial equivalent;

digestion with BglII and detection of a 24 kb fragment using renin 5' probe, renin 3' probe, or a substantial equivalent;

digestion with RsaI and detection of a 0.9 kb fragment using renin 5' probe, renin 3' probe, or a substantial equivalent;

digestion with HindIII and detection of a 6.2 kb fragment using renin 3'probe or a substantial equivalent;

digestion with TaqI and detection of an 11.0 kb fragment using renin 5' probe, renin 3' probe, or a substantial equivalent;

digestion with EcoRI and detection of a 3.9kb fragment using a mixture of renin 5' and renin 3' probe or a substantial equivalent;

digestion with BanII and detection of a 1.4 kb fragment using ANF "3-oligo" mixture probe, or a substantial equivalent;

digestion with BglI and detection of a 4.1 kb fragment using ANF "3-oligo" mixture probe, or a substantial equivalent;

digestion with BglII and detection of a 9.4 kb fragment using ANF "3-oligo" mixture probe, or a substantial equivalent;

digestion with EcoRI and detection of a 5.2 kb fragment using ANF "3-oligo" mixture probe, or a substantial equivalent;

digestion with EcoRI and detection of a 15 kb fragment using HK1 probe or a substantial equivalent.

digestion with PstI and detection of a 3.1 kb fragment using λHK1 probe or a substantial equivalent.

digestion with StuI and detection of a 4.5 kb fragment using λHK1 probe or a substantial equivalent.

3. A method of genetically identifying an individual, which method comprises assessing the presence or absence of polymorphisms in a series of genomic DNA digests

wherein said assessment is conducted by at least one of the processes selected from

probing with renin 5' probe, or with a substantial equivalent, a digest obtained by treatment of a human gene library with at least one of BglI, BglII, RsaI, EcoRI, or TaqI;

probing with renin 3' probe, or with a substantial equivalent, a digest obtained by treatment of a human genomic library with at least one of BglII, HindII, RsaI, EcoRI or TaqI;

probing with ANF "3-oligo" mixture probe, or with a substantial equivalent, a digest obtained by treatment with at least one of BanII, BglI, BglII, or EcoRI;

probing with λHK1 probe, or with a substantial equivalent, a said digest are obtained by treatment of a human genomic library with at least one of EcoRI, PstI, or StuI.

4. A reagent kit useful in performing the method of claim 1, which kit includes at least one of renin 5' probe, renin 3' probe, ANF "3-oligo" mixture probe, λHK1 probe, or substantial equivalent and at least one suitable restriction enzyme selected from the group consisting of BglI, BglII, BanII, HindIII, RsaI, TaqI, EcoRI, PstI, and StuI.

5. A recombinant DNA encoding human kallikrein substantially free of DNA encoding other human kidney proteins.

6. The DNA of claim 5 wherein the human kallikrein has the amino acid sequence shown in Figure 3.

7. An expression system capable, when contained in a suitable recombinant host cell, of effecting the production of human kallikrein which comprises the DNA of claim 5.

8. Recombinant host cells which contain the DNA of claim 5.

9. Human kallikrein produced by the cells of claim 8.

10. A method for producing human kallikrein which comprises culturing the cells of claim 8.

```
                      -42  AACCTCAGTGGATCTCACAGAGA    CAGACTGAGGGAAGC   -1

  1  ATG GAT GGA TGG AGA AGG ATG CCT CGC TGG GGA CTG CTG CTG CTG CTC TGG GGC TCC TGT   60
     Met Asp Gly Trp Arg Arg Met Pro Arg Trp Gly Leu Leu Leu Leu Leu Trp Gly Ser Cys
      1                  {-60}          10                    {-50}            20

 61  ACC TTT GGT CTC CCG ACA GAC ACC ACC ACC TTT AAA CGG ATC TTC CTC AAG AGA ATG CCC  120
     Thr Phe Gly Leu Pro Thr Asp Thr Thr Thr Phe Lys Arg Ile Phe Leu Lys Arg Met Pro
                      {-40}           30                    {-30}              40

121  TCA ATC CGA GAA AGC CTG AAG GAA CGA GGT GTG GAC ATG GCC AGG CTT GGT CCC GAG TGG  180
     Ser Ile Arg Glu Ser Leu Lys Glu Arg Gly Val Asp Met Ala Arg Leu Gly Pro Glu Trp
                      {-20}           50                    {-10}              60

181  AGC CAA CCC ATG AAG AGG CTG ACA CTT GGC AAC ACC ACC TCC TCC GTG ATC CTC ACC AAC  240
     Ser Gln Pro Met Lys Arg Leu Thr Leu Gly Asn Thr Thr Ser Ser Val Ile Leu Thr Asn
                      {-1}{1}          70                    {10}              80

241  TAC ATG GAC ACC CAG TAC TAT GGC GAG ATT GGC ATC GGC ACC CCA CCC CAG ACC TTC AAA  300
     Tyr Met Asp Thr Gln Tyr Tyr Gly Glu Ile Gly Ile Gly Thr Pro Pro Gln Thr Phe Lys
                      {20}            90                    {30}             100

301  GTC GTC TTT GAC ACT GGT TCG TCC AAT GTT TGG GTG CCC TCC TCC AAG TGC AGC CGT CTC  360
     Val Val Phe Asp Thr Gly Ser Ser Asn Val Trp Val Pro Ser Ser Lys Cys Ser Arg Leu
                      {40}           110                    {50}             120

361  TAC ACT GCC TGT GTG TAT CAC AAG CTC TTC GAT GCT TCG GAT TCC TCC AGC TAC AAG CAC  420
     Tyr Thr Ala Cys Val Tyr His Lys Leu Phe Asp Ala Ser Asp Ser Ser Ser Tyr Lys His
                      {60}           130                    {70}             140

421  AAT GGA ACA GAA CTC ACC CTC CGC TAT TCA ACA GGG ACA GTC AGT GGC TTT CTC AGC CAG  480
     Asn Gly Thr Glu Leu Thr Leu Arg Tyr Ser Thr Gly Thr Val Ser Gly Phe Leu Ser Gln
                      {80}           150                    {90}             160

481  GAC ATC ATC ACC GTG GGT GGA ATC ACG GTG ACA CAG ATG TTT GGA GAG GTC ACG GAG ATG  540
     Asp Ile Ile Thr Val Gly Gly Ile Thr Val Thr Gln Met Phe Gly Glu Val Thr Glu Met
                      {100}          170                    {110}            180

541  CCC GCC TTA CCC TTC ATG CTG GCC GAG TTT GAT GGG GTT GTG GGC ATG GGC TTC ATT GAA  600
     Pro Ala Leu Pro Phe Met Leu Ala Glu Phe Asp Gly Val Val Gly Met Gly Phe Ile Glu
                      {120}          190                    {130}            200

601  CAG GCC ATT GGC AGG GTC ACC CCT ATC TTC GAC AAC ATC ATC TCC CAA GGG GTG CTA AAA  660
     Gln Ala Ile Gly Arg Val Thr Pro Ile Phe Asp Asn Ile Ile Ser Gln Gly Val Leu Lys
                      {140}          210                    {150}            220

661  GAG GAC GTC TTC TCT TTC TAC TAC AAC AGA GAT TCC GAG AAT TCC CAA TCG CTG GGA GGA  720
     Glu Asp Val Phe Ser Phe Tyr Tyr Asn Arg Asp Ser Glu Asn Ser Gln Ser Leu Gly Gly
                      {160}          230                    {170}            240

721  CAG ATT GTG CTG GGA GGC AGC GAC CCC CAG GAT TAC GAA GGG AAT TTC CAC TAT ATC AAC  780
     Gln Ile Val Leu Gly Gly Ser Asp Pro Gln Asp Tyr Glu Gly Asn Phe His Tyr Ile Asn
                      {180}          250                    {190}            260

781  CTC ATC AAG ACT GGT GTC TGG CAG ATT CAA ATG AAG GGG GTG TCT GTG GGG TCA TCC ACC  840
     Leu Ile Lys Thr Gly Val Trp Gln Ile Gln Met Lys Gly Val Ser Val Gly Ser Ser Thr
                      {200}          270                    {210}            280

841  TTG CTC TGT GAA GAC GGC TGC CTG GCA TTG GTA GAC ACC GGT GCA TCC TAC ATC TCA GGT  900
     Leu Leu Cys Glu Asp Gly Cys Leu Ala Leu Val Asp Thr Gly Ala Ser Tyr Ile Ser Gly
                      {220}          290                    {230}            300

901  TCT ACC AGC TCC ATA GAG AAG CTC ATG GAG GCC TTG GGA GCC AAG AAG AGG CTG TTT GAT  960
     Ser Thr Ser Ser Ile Glu Lys Leu Met Glu Ala Leu Gly Ala Lys Lys Arg Leu Phe Asp
                      {240}          310                    {250}            320

961  TAT GTC GTG AAG TGT AAC GAG GGC CCT ACA CTC CCC GAC ATC TCT TTC CAC CTG GGA GGC 1020
     Tyr Val Val Lys Cys Asn Glu Gly Pro Thr Leu Pro Asp Ile Ser Phe His Leu Gly Gly
                      {260}          330                    {270}            340

1021 AAA GAA TAC ACG CTC ACC AGC GCG GAC TAT GTA TTT CAG GAA TCC TAC AGT AGT AAA AAG 1080
     Lys Glu Tyr Thr Leu Thr Ser Ala Asp Tyr Val Phe Gln Glu Ser Tyr Ser Ser Lys Lys
                      {280}          350                    {290}            360

1081 CTG TGC ACA CTG GCC ATC CAC GCC ATG GAT ATC CCG CCA CCC ACT GGA CCC ACC TGG GCC 1140
     Leu Cys Thr Leu Ala Ile His Ala Met Asp Ile Pro Pro Pro Thr Gly Pro Thr Trp Ala
                      {300}          370                    {310}            380

1141 CTG GGG GCC ACC TTC ATC CGA AAG TTC TAC ACA GAG TTT GAT CGG CGT AAC AAC CGC ATT 1200
     Leu Gly Ala Thr Phe Ile Arg Lys Phe Tyr Thr Glu Phe Asp Arg Arg Asn Asn Arg Ile
                      {320}          390                    {330}            400

1201 GGC TTC GCC TTG GCC CGC TGAGGCCCTCTGCCACCCAGGCAGGCCCTGCCTTCAGCCCTGGCCCAGAGCTGGA 1273
     Gly Phe Ala Leu Ala Arg
                      {340}406

1274 ACACTCTCTGAGATGCCCCTCTGCCTGGGCTTATGCCCTCAGATGGAGACATTGGATGTGGAGCTCCTGCTGGATGCGT 1352

1353 GCCCTGACCCCTGCACCAGCCCTTCCCTGCTTTGAGGACAAAGAGAATAAAGACTTCATGTTCAC
```

# FIG. 1

[from Imai et al., Proc. Nat. Acad. Sci. USA 80:7405-7409 (1983)]

PROBE SEQUENCE 1

ggatccatttgtctcgggctgctggctgcctgccatttcctcctctccaccct

PROBE SEQUENCE 2

CTCAGCCCCCTCCCTGAGGTGCCTCCCTGGACCGGGGAAGTCAGCCCAGCCCA

PROBE SEQUENCE 3

taccatggacagaagacaaatgcctgcgttggtgtgctttctttcttcttgggaagagaattc

FIGURE 2

```
                                        -1  1                                    10
          Ala Pro Pro Ile Gln Ser Arg Ile Val Gly Gly Trp Glu Cys Glu Gln His
          GCG CCC CCG AUU CAG UCC CGG AUU GUG GGA GGC UGG GAG UGU GAG CAG CAU

                                        20                                       30
  Ser Gln Pro Trp Gln Ala Ala Leu Tyr His Phe Ser Thr Phe Gln Cys Gly Gly Ile Leu
  UCC CAG CCC UGG CAG GCG GCU CUG UAC CAU UUC AGC ACU UUC CAG UGU GGG GGC AUC CUG

                                        40                                       50
  Val His Arg Gln Trp Val Leu Thr Ala Ala His Cys Ile Ser Asp Asn Tyr Gln Leu Trp
  GUG CAC CGC CAG UGG GUG CUC ACA GCU GCU CAU UGC AUC AGC GAC AAU UAC CAG CUC UGG

                                        60                                       70
  Leu Gly Arg His Asn Leu Phe Asp Asp Glu Asn Thr Ala Gln Phe Val His Val Ser Glu
  CUG GGU CGC CAC AAC UUG UUU GAC GAC GAA AAC ACA GCC CAG UUU GUU CAU GUC AGU GAG

                                        80                                       90
  Ser Phe Pro His Pro Gly Phe Asn Met Ser Leu Leu Glu Asn His Thr Arg Gln Ala Asp
  AGC UUC CCA CAC CCU GGC UUC AAC AUG AGC CUC CUG GAG AAC CAC ACC CGC CAA GCA GAC

                                        100                                      110
  Glu Asp Tyr Ser His Asp Leu Met Leu Leu Arg Leu Thr Glu Pro Ala Asp Thr Ile Thr
  GAG GAC UAC AGC CAC GAC CUC AUG CUG CUC CGC CUG ACA GAG CCU GCU GAU ACC AUC ACA

                                        120                                      130
  Asp Ala Val Lys Val Val Glu Leu Pro Thr Gln Glu Pro Glu Val Gly Ser Thr Cys Leu
  GAC GCU GUG AAG GUC GUG GAG UUG CCC ACC CAG GAA CCC GAA GUG GGG AGC ACC UGU UUG

                                        140                                      150
  Ala Ser Gly Trp Gly Ser Ile Glu Pro Glu Asn Phe Ser Phe Pro Asp Asp Leu Gln Cys
  GCU UCC GGC UGG GGC AGC AUC GAA CCA GAG AAU UUC UCA UUU CCA GAU GAU CUC CAG UGU

                                        160                                      170
  Val Asp Leu Lys Ile Leu Pro Asn Asp Glu Cys Glu Lys Ala His Val Gln Lys Val Thr
  GUG GAC CUC AAA AUC CUG CCU AAU GAU GAG UGC GAA AAA GCC CAC GUC CAG AAG GUG ACA

                                        180              *                       190
  Asp Phe Met Leu Cys Val Gly His Leu Glu Gly Gly Lys Asp Thr Cys Val Gly Asp Ser
  GAC UUC AUG CUG UGU GUC GGA CAC CUG GAA GGU GGC AAA GAC ACC UGU GUG GGU GAU UCA

                                        200                                      210
  Gly Gly Pro Leu Met Cys Asp Gly Val Leu Gln Gly Val Thr Ser Trp Gly Tyr Val Pro
  GGG GGC CCG CUG AUG UGU GAU GGU GUG CUC CAA GGU GUC ACA UCA UGG GGC UAC GUC CCU

                                        220                                      230
  Cys Gly Thr Pro Asn Lys Pro Ser Val Ala Val Arg Val Leu Ser Tyr Val Lys Trp Ile
  UGU GGC ACC CCC AAU AAG CCU UCU GUC GCC GUC AGA GUG CUG UCU UAU GUG AAG UGG AUC

                                        238
  Glu Asp Thr Ile Ala Glu Asn Ser
  GAG GAC ACC AUA GCG GAG AAC UCC UGA
```

FIG. 3

15 DNAs digested with BglI and hybridized to the 5' cDNA probe

FIG. 4